(19) Europäisches Patentamt
European Patent Office
Office européen des brevets

(11) Veröffentlichungsnummer: **0 193 492 B1**

# (12) EUROPÄISCHE PATENTSCHRIFT

(45) Veröffentlichungstag der Patentschrift: **24.08.88**

(51) Int. Cl.⁴: **C 07 C 143/66**

(21) Anmeldenummer: **86810073.6**

(22) Anmeldetag: **10.02.86**

## (54) Verfahren zur Herstellung von 1-Amino-2-naphthol-4-sulfonsäure.

(30) Priorität: **15.02.85 CH 731/85**

(43) Veröffentlichungstag der Anmeldung: **03.09.86 Patentblatt 86/36**

(45) Bekanntmachung des Hinweises auf die Patenterteilung: **24.08.88 Patentblatt 88/34**

(84) Benannte Vertragsstaaten: **CH DE FR GB IT LI**

(56) Entgegenhaltungen:
**EP - A - 0 080 644**

**CHEMICAL ABSTRACTS, Band 64, Nr. 11, 23. Mai 1966, Nr. 15813h, Columbus, Ohio, US; & CS - A - 115 051 (J. ZALOUDEK et al.) 15-06-1965**

(73) Patentinhaber: **CIBA-GEIGY AG, Klybeckstrasse 141, CH-4002 Basel (CH)**

(72) Erfinder: **Förtsch, Bruno, Dr., Eggweg, CH-4433 Ramlinsburg (CH)**

EP 0 193 492 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung von 1-Amino-2-naphthol-4-sulfonsäure durch Behandeln von 1-Hydroxylimino-2-tetralon-4-sulfonsäure mit Pyrosulfit in mineralsaurem Medium.

1-Amino-2-naphthol-4-sulfonsäure ist ein wichtiges Zwischenprodukt zur Herstellung von Textilfarbstoffen (siehe z.B. N.N. Woroshzow «Grundlagen der Synthese von Zwischenprodukten und Farbstoffen» Akademie-Verlag, Berlin 1966). Hergestellt wird diese Verbindung allgemein ausgehend von β-Naphthol, das in α-Stellung nitrosiert und anschliessend mit Hydrogensulfit zur 1-Hydroxylimino-2-tetralon-4-sulfonsäure umgesetzt wird, die in Gegenwart eines Überschusses an Hydrogensulfit in schwefelsaurer Lösung zur 1-Amino-2-naphthol-4-sulfonsäure weiterreagiert [Ullmanns Enzyklopädie der technischen Chemie, 4. Auflage, Band 17 (1979) Seite 100].

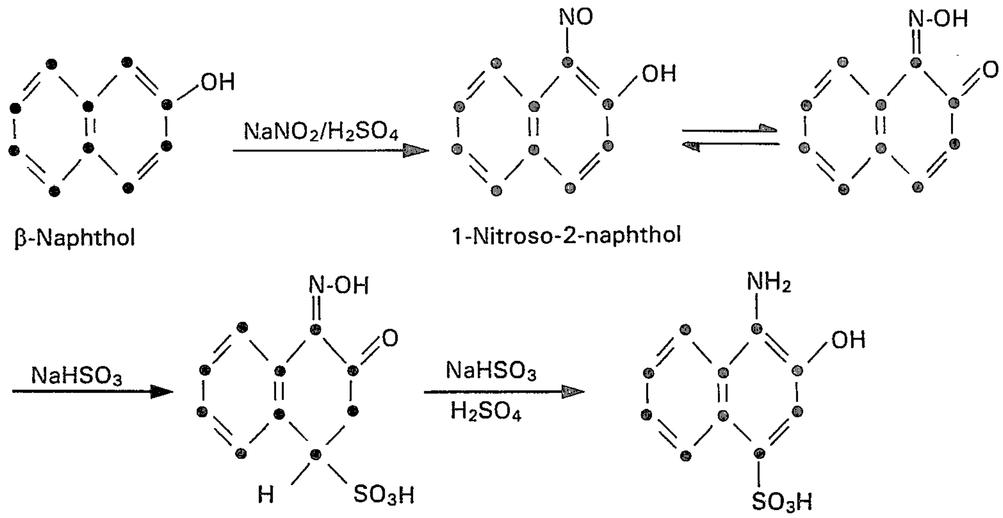


β-Naphthol

1-Nitroso-2-naphthol

1-Hydroxylimino-2-tetralon-4-sulfonsäure

1-Amino-2-naphthol-4-sulfonsäure

Hinsichtlich der letzten Stufe dieser Reaktionsfolge, der Reduktion der 1-Hydroxylimino-2-tetralon-4-sulfonsäure zur Aminonaphtholsulfonsäure lässt sich eine beträchtliche Ausbeutesteigerung erzielen, wenn man dem Reaktionsgemisch ein Eisensalz zusetzt (EP-A-0 080 644). Nachteil dieses Verfahrens ist jedoch, dass relativ grosse Mengen an Eisensalz benötigt werden, um die gewünschte Ausbeutesteigerung zu erreichen und zwar etwa 0,5 bis 1 Mol Eisen-II-sulfat auf 1 Mol Hydroxyliminotetralonsulfonsäure bzw. β-Naphthol. Derartige Mengen an Eisensalz führen nach der Aufarbeitung zu einer erheblichen Abwasserbelastung.

Aufgabe der vorliegenden Erfindung ist es daher ein Verfahren zur Reduktion der 1-Hydroxylimino-2-tetralon-4-sulfonsäure zu entwickeln, das in guter Ausbeute eine möglichst reine 1-Amino-2-naphthol-4-sulfonsäure liefert, dabei jedoch ohne grössere Mengen an Schwermetallsalzen auskommt.

Gefunden wurde, dass die Umwandlung der Hydroxyliminotetralonsulfonsäure in die Aminonaphtholsulfonsäure auch durch Kupferverbindungen katalysiert wird, wobei diese überraschenderweise bereits in deutlich geringerer Konzentration wirksam sind, als die bisher eingesetzten Eisensalze.

Gegenstand der vorliegenden Erfindung ist somit ein Verfahren zur Herstellung von 1-Amino-2-naphthol-4-sulfonsäure durch Behandeln von 1-Hydroxylimino-2-tetralon-4-sulfonsäure mit einem Alkalimetallpyrosulfit in mineralsaurem Medium, das dadurch gekennzeichnet ist, dass man die Reaktion in Gegenwart katalytischer Mengen einer Kupferverbindung durchführt.

Die 1-Hydroxylimino-2-tetralon-4-sulfonsäure kann sowohl als freie Säure, wie auch in Form eines Alkalimetallsalzes, z.B. des Natrium- oder Kaliumsalzes eingesetzt werden. Nach dem vorstehend beschriebenen Reaktionsweg, ausgehend von β-Naphthol, ohne Isolieren der Zwischenstufen, erhält man eine wässrige Alkalimetallsalzlösung der 1-Hydroxylimino-2-tetralon-sulfonsäure, die in dieser Form direkt zur Aminonaphtholsulfonsäure reduziert werden kann. Eine vorhergehende Isolierung der Hydroxyliminotetralonsulfonsäure ist nicht nötig, es empfiehlt sich jedoch, deren wässrige Lösung vor der Weiterverarbeitung einer Klärfiltration zu unterwerfen. Die Reaktion kann kontinuierlich oder diskontinuierlich durchgeführt werden.

Die Reduktion wird in mineralsaurer Lösung durchgeführt, vorteilhaft arbeitet man in Schwefelsäure einer Konzentration von 40 bis 70%. Anstelle von Schwefelsäure kann beispielsweise auch Salzsäure verwendet werden.

Als Reduktionsmittel verwendet man ein Alkalimetallpyrosulfit, z.B. Natrium- oder Kaliumpyrosulfit, das im Reaktionsgemisch mit dem entsprechenden Hydrogensulfit im Gleichgewicht steht. Es versteht sich daher von selbst, dass anstelle der Pyrosulfite auch Hydrogensulfite verwendet werden können. Wichtig ist, dass die Reaktion unter Ausschluss von Luftsauerstoff durchgeführt wird, damit es nicht zu einer Reoxidation der Aminonaphtholsulfonsäure kommt.

Erfindungswesentlich ist, dass die Reaktion in Gegenwart katalytischer Mengen einer Kupferverbindung durchgeführt wird. Als Kupferverbindungen

kommen beispielsweise Kupfersalze, Kupferhydroxide, Kupferoxide oder auch Kupferkomplexe in Frage. Als Salze z.B. Sulfate, Nitrate, Chloride, Bromide, Jodide, Carbonate, Formiate, Acetate oder Phosphate; als Hydroxide und Oxide z.B. Kupfer-II-oxidhydrat, Kupfer-I-oxid oder Kupfer-II-oxid. Bevorzugt verwendet werden Kupfer-II-salze, insbesondere das Kupfer-II-sulfat. Die Kupferverbindungen können sowohl in fester Form als auch in gelöster Form, z.B. als wässrige oder schwefelsaure Lösung verwendet werden.

Bezogen auf 1 Mol zu reduzierende 1-Hydroxylimino-2-tetralon-sulfonsäure verwendet man zweckmässigerweise 0,5 bis 50 mMol, insbesondere 1 bis 10 mMol an Kupferverbindung. Mengen über 50 mMol Kupferverbindung pro Mol Hydroxylimino-2--tetralonsulfonsäure führen zu keiner wesentlichen Steigerung der Raum-Zeit-Ausbeute, vielmehr kann es zu einer Verminderung der Ausbeute kommen. Hingegen sollte der Grenzwert von 0,5 mMol nicht unterschritten werden, da dies zu einem deutlichen Nachlassen des katalytischen Effekts führt.

Durchgeführt wird die Reduktion bei einer Temperatur die zweckmässigerweise im Bereich von 20° bis 100°C, insbesondere zwischen 40° und 60°C liegt. Die Reaktionszeit beträgt ca. 1/2 bis 2 Stunden. Bei Temperaturen $\geqslant$ 60°C wird in der Regel unter Druck gearbeitet. Je nach Reaktionstemperatur baut sich ein Druck von bis zu 5 atm auf.

Um die 1-Amino-2-naphthol-4-sulfonsäure in einer gut filtrierbaren Form zu erhalten, erweist es sich als vorteilhaft, das während der Reaktion in kristalliner Form ausgefallene Produkt nicht sofort aus dem Reaktionsgemisch zu isolieren, sondern zuvor einer thermischen Nachbehandlung zu unterwerfen. Durch diese Massnahme wird der kolloiddisperse Anteil verringert, was sich in kürzeren Filtrationszeiten und einem geringeren Wassergehalt des Filterkuchens deutlich bemerkbar macht. Ein sehr gut filtrierbares Produkt bekommt man, wenn das ausreagierte Reaktionsgemisch 5 bis 15 Stunden, insbesondere 8 bis 12 Stunden unter Rühren auf eine Temperatur von 40° bis 60°C, insbesondere 45° bis 50°C erhitzt wird.

Die Abtrennung der kristallinen 1-Amino-2-naphthol-4-sulfonsäure aus dem Reaktionsgemisch erfolgt auf bekannte Art und Weise, z.B. durch Filtrieren oder Zentrifugieren. Anschliessend wird das Produkt gegebenenfalls neutral gewaschen und getrocknet. Das Produkt kann natürlich auch in Form des feuchten Filterkuchens direkt weiterverarbeitet werden, z.B. durch Diazotieren zur Diazonaphtholsulfonsäure.

Das wichtigste Folgeprodukt der 1-Amino-2-naphthol-4-sulfonsäure ist die 1-Diazo-2-naphthol-4-sulfonsäure, die als Azokomponente zur Herstellung einer Vielzahl von Textil- und auch Lederfarbstoffen dient, insbesondere zur Herstellung von Metallkomplexfarbstoffen für die Wollfärberei.

Das folgende Beispiel dient der Veranschaulichung der Erfindung; Teile bedeuten Gewichtsteile und Prozente Gewichtsprozente.

*Beispiel*

In einem Reaktor werden 80 Teile Wasser, 77,2 Teile 45%ige Schwefelsäure und 0,15 Teile Kupfer-II-sulfat ($Cu\ SO_4 \times 5\ H_2O$) vorgelegt. Der Kessel wird geschlossen und mit Stickstoff gespült. Dann werden 1,38 Teile Natriumpyrosulfit gelöst in 4 Teilen Wasser zugegeben und der Kesselinhalt wird auf eine Temperatur von 45°C erhitzt. Ist die Temperatur erreicht, werden innerhalb von 2 Stunden bei einer Innentemperatur von 45° bis 50°C Teile einer ca. 12%igen wässrigen Lösung von 1-Hydroxylimino-2-tetralon-sulfonsäure — erhalten durch Nitrosieren von 21,6 Teilen β-Naphthol und anschliessende Bisulfitaddition — zugegeben. Man erhält eine Kristallsuspension, die man noch weitere 10 Stunden bei einer Temperatur von 45° bis 50°C rührt. Danach wird das Produkt abfiltriert, neutral gewaschen und getrocknet. Man erhält 30,2 Teile 1-Amino-2-naphthol-4-sulfonsäure mit einem Gehalt von 97,1%, das entspricht einer Ausbeute von 81,7%, berechnet auf β-Naphthol. $R_f$ = 0,3 (Kieselgelplatte mit UV Indikator). Der $R_f$-Wert stimmt mit dem einer authentischen Probe überein.

Ein vergleichbar gutes Ergebnis erhält man, wenn man anstelle des Kupfer-II-sulfats die entsprechende Menge an Kupfer-II-chlorid oder Kupfer-II-oxidhydrat verwendet. Wird die hier eingesetzte Menge von ca. 5 mMol Kupfer-II-salz pro Mol 1-Hydroxylimino-2-tetralon-sulfonsäure auf 10 bzw. 20 mMol pro Mol Tetralonsulfonsäure erhöht, so liegen die erzielten Ausbeuten an 1-Amino-2-naphthol-4-sulfonsäure im Bereich von 80 bis 85%.

## Patentansprüche

1. Verfahren zur Herstellung von 1-Amino-2--naphthol-4-sulfonsäure durch Behandeln von 1-Hydroxylimino-2-tetralon-sulfonsäure mit einem Alkalimetall-pyrosulfit in mineralsaurem Medium, dadurch gekennzeichnet, dass man die Reaktion in Gegenwart katalytischer Mengen einer Kupferverbindung durchführt.

2. Verfahren gemäss Anspruch 1, dadurch gekennzeichnet, dass man als Kupferverbindung ein Kupfer-II-salz verwendet.

3. Verfahren gemäss Anspruch 1, dadurch gekennzeichnet, dass man bezogen auf 1 Mol 1-Hydroxylimino-2-tetralon-sulfonsäure 0,5 bis 50 mMol an Kupferverbindung einsetzt.

4. Verfahren gemäss Anspruch 3, dadurch gekennzeichnet, dass man bezogen auf 1 Mol 1-Hydroxylimino-2-tetralon-sulfonsäure 1 bis 10 mMol an Kupferverbindung einsetzt.

5. Verfahren gemäss Anspruch 1, dadurch gekennzeichnet, dass das Produkt einer thermischen Nachbehandlung unterworfen wird.

6. Verfahren gemäss Anspruch 5, dadurch gekennzeichnet, dass das Reaktionsgemisch vor Isolieren der 1-Amino-2-naphthol-4-sulfonsäure während 5 bis 15 Stunden, insbesondere 8 bis 12 Stunden unter Rühren auf eine Temperatur von 40° bis 60°C, insbesondere 45° bis 50°C erhitzt wird.

## Claims

1. A process for the preparation of 1-amino-2--naphthol-4-sulfonic acid by treating 1-hydroxylimino-2-tetralone-sulfonic acid with an alkali metal pyrosulfite in mineral acid medium, which process comprises carrying out the reaction in the presence of catalytic amounts of a copper compound.

2. A process according to claim 1, wherein the copper compound is a copper(II) salt.

3. A process according to claim 1, wherein 0.5 to 50 millimoles of copper compound are used per mole of 1-hydroxylimino-2-tetralone-4-sulfonic acid.

4. A process according to claim 3, wherein 1 to 10 millimoles of copper compound are used per mole of 1-hydroxylimino-2-tetralone-4-sulfonic acid.

5. A process according to claim 1, wherein the product is subjected to a thermal aftertreatment.

6. A process according to claim 5, wherein the reaction mixture is heated for 5 to 15 hours, in particular for 8 to 12 hours, with stirring, to a temperature from 40° to 60°C, in particular from 45° to 50°C, before isolation of the 1-amino-2-naphthol-4-sulfonic acid.

**Revendications**

1. Procédé pour la préparation d'acide 1-amino-2--naphtol-4-sulfonique, par traitement de l'acide 1-hydroxylimino-2-tétralone-4-sulfonique avec un pyrosulfite de métal alcalin en milieu acidifié par un acide minéral, caractérisé par le fait que l'on effectue la réaction en présence de quantités catalytiques d'un composé à base de cuivre.

2. Procédé selon la revendication 1, caractérisé par le fait qu'en tant que composé à base de cuivre, on utilise un sel cuivrique.

3. Procédé selon la revendication 1, caractérisé par le fait que l'on utilise 0,5 à 50 mmoles de composé à base de cuivre, par rapport à 1 mole d'acide 1-hydroxylimino-2-tétralone-4-sulfonique.

4. Procédé selon la revendication 3, caractérisé par le fait que l'on utilise 1 à 10 mmoles de composé à base de cuivre, par rapport à 1 mole d'acide 1-hydroxylimino-2-tétralone-4-sulfonique.

5. Procédé selon la revendication 1, caractérisé par le fait que l'on soumet le produit à un post-traitement thermique.

6. Procédé selon la revendication 5, caractérisé par le fait qu'avant l'isolement de l'acide 1-amino-2--naphtol-4-sulfonique, on chauffe le mélange réactionnel pendant 5 à 15 heures, en particulier 8 à 12 heures, sous agitation, à une température de 40° à 60°C, en particulier de 45° à 50°C.